(19) Europäisches Patentamt / European Patent Office / Office européen des brevets

(11) **EP 2 153 812 B1**

(12) **EUROPÄISCHE PATENTSCHRIFT**

(45) Veröffentlichungstag und Bekanntmachung des Hinweises auf die Patenterteilung:
**26.11.2014 Patentblatt 2014/48**

(51) Int Cl.:
*A61K 49/04* *(2006.01)*  *B82Y 5/00* *(2011.01)*
*A61K 6/00* *(2006.01)*

(21) Anmeldenummer: **08014437.1**

(22) Anmeldetag: **13.08.2008**

(54) **Röntgenopaker Infiltrant**

X-ray opaque infiltrant

Infiltrant radio-opaque

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(43) Veröffentlichungstag der Anmeldung:
**17.02.2010 Patentblatt 2010/07**

(60) Teilanmeldung:
**12188131.2 / 2 548 546**

(73) Patentinhaber: **Mühlbauer Technology GmbH**
**22547 Hamburg (DE)**

(72) Erfinder:
• **Neffgen, Stephan, Dr.**
  **22459 Hamburg (DE)**
• **Neander, Swen, Dr.**
  **20148 Hamburg (DE)**
• **Lübbers, Dierk, Dr.**
  **25469 Halstenbek (DE)**

(74) Vertreter: **Glawe, Delfs, Moll**
**Partnerschaft mbB von**
**Patent- und Rechtsanwälten**
**Rothenbaumchaussee 58**
**20148 Hamburg (DE)**

(56) Entgegenhaltungen:
EP-A- 1 570 831    EP-A- 1 849 450
EP-A- 1 872 767    WO-A1-2007/131725
DE-A1- 19 638 068

**Beschreibung**

Die Erfindung betrifft einen Infiltranten für die Dentalapplikation, der vernetzende Monomere enthält sowie dessen Verwendung zur Prävention bzw. Behandlung (Versiegelung) käriöser Schmelzläsionen.

Unter käriösen Schmelzläsionen sind hier im Wesentlichen sich im Zahnschmelz ausdehnende kariöse Schädigungen zu verstehen, die noch nicht zu Kavitation (Lochbildung) geführt haben. Kariöse Schmelzläsionen sind demineralisierte Bereiche des Zahnschmelzes, die eine Tiefe bis zu 2-3 mm haben können.

Aus der veröffentlichten Internationalen Anmeldung WO 2007/131725 A1 sind zur Behandlung kariöser Schmelzläsionen ein Infiltrationsverfahren und Infiltranten bekannt, so dass die Kavitation verhindert wird und sich die sonst üblicherweise erfolgende Restauration mit Dentalkompositen erübrigt. Bei dem Infiltrationsverfahren wird, nachdem eine evtl. vorhandene remineralisierte oberflächliche Schicht entfernt wurde, die Läsion mit einem Infiltranten, der im Wesentlichen aus Monomeren besteht, in Kontakt gebracht, woraufhin diese infiltrieren. Nachdem der Infiltrant die Läsion durchdrungen hat, werden die Monomere mittels Lichtaktivierung polymerisiert. Die Läsion ist dann versiegelt. Das Fortschreiten der Karies wird gestoppt.

Für die Infiltration sind spezielle Monomere bzw. Monomermischungen erforderlich, da bekannte Dentalkleber für Dentalkomposite (auch Adhäsive oder Bondings genannt) zu langsam und/oder nicht ausreichend in die Läsion eindringen und/oder die Läsion vollständig durchdringen (penetrieren oder infiltrieren). Die WO 2007/131725 A1 beschreibt die Verwendung Monomerer bzw. Monomermischungen, so dass der Infiltrant einen Penetrationskoeffizienten PK > 50 hat.

Von Infiltranten zu unterscheiden sind Zahnrestaurationscompomere, wie in der DE 19638068 A1 beschrieben, die eine möglichst starke Haftung zur Zahnsubstanz aufweisen sollen und ein dementsprechend geringes Penetrationsvermögen haben.

Der Erfindung liegt die Aufgabe zugrunde, einen Infiltranten der eingangs genannten Art zu schaffen, der eine einfache Erfolgskontrolle einer durchgeführten Infiltration ermöglicht.

Die Erfindung löst die Aufgabe mit einem Infiltrant gemäß Anspruch 1. Vorteilhafte Ausführungsformen der Erfindung sind in den Unteransprüchen offenbart.

Um zu vorliegender Erfindung zu gelangen sind für den Fachmann nachfolgend beschriebene Schritte a) und b) erforderlich.

a) Erkennen des Nachteils der aus dem Stand der Technik bekannten Infiltranten (unzureichende Röntgenopazität)

Nachteilig an den aus dem Stand der Technik (z.B. WO 2007/131725 A1) bekannten Infiltranten ist deren unzureichende Röntgenopazität. Sie sind für Röntgenstrahlen im Wesentlichen durchlässig (transluzent) und daher bei einer Röntgendiagnostik nicht oder nur sehr schwer erkennbar. Damit ist eines der wichtigsten Instrumente zur Erkennung des Ausmaßes und der Lage vorhandener Infiltrationen entwertet. Abgesehen davon ist es bei Einsatz der Röntgendiagnostik schwierig auf Grund der unzureichenden Röntgenopazität der Infiltranten eine evtl. unter der infiltrierten Läsion weiter fortschreitende Karies festzustellen, da sie nicht oder sehr schlecht vom infiltrierten Bereich unterschieden werden kann. Um eine fortschreitende Karies festzustellen, sind dann aufwendige genau reproduzierbare Bissflügelaufnahmen erforderlich, wie sie in der deutschen Gebrauchsmusteranmeldung DE 202008006814.2 beschrieben sind.

Die Erfindung hat in einem ersten Schritt erkannt, dass durch Infiltration restaurierte Zahnbereiche mittels Röntgendiagnostik nicht leicht zu identifizieren sind und möglicherweise durch eine höhere Röntgenopazität des Infiltrants ein für die röntgendiagnostische Untersuchung ausreichender Kontrast zum umgebenden gesunden Zahn- und Knochengewebe undd/oder auch zu kariösem Zahn- und Knochengewebe hergestellt werden könnte.

b) Bereitstellung eines geeigneten Infiltrant-Kontrastmittels unter Überwindung eines Vorurteils der Fachwelt

Die unzureichende Kontrastierung, wie sie für Infiltrationen festgestellt wurde, stellt sich bei den herkömmlich verwendeten Dentalmaterialien, wie sie bei der Restauration oder dem Zahnersatz zu Einsatz kommen, nicht. Die im Stand der Technik verwendeten metallischen Restaurationen erzeugen bereits von sich aus einen guten Kontrast. Gleiches gilt für keramische Materialien und Kunststoffkomposite, bei denen die vorrangig aus Gründen der Erhöhung der mechanischen Festigkeit und der Reduzierung des Schrumpfs zugesetzten Füllstoffe und/oder Pigmente einen ausreichenden röntgenopaken Kontrast bereitstellen. Beispielsweise offenbaren die EP 1570831 A1 und die EP 1872767 polymerisierbare Dentalmaterialen, denen zur Verbesserung der mechanischen Eigenschaften, u.a. röntgenopake Füllstoffe zugegeben werden.

Geeignete Gläser und nanoskalige Füllstoffe und Füllstoffkombinationen für Dentalkomposite sind dem Fachmann bekannt und bspw. in der EP 1720506 A1 beschrieben.

Nanoskalige Füllstoffe hatte der Fachmann auf dem Gebiet der vorliegenden Erfindung, insbesondere der Fachmann nach WO 2007/131725 nicht als Komponente eines Infiltrants in Betracht gezogen. Zum ersten nicht, weil er das unter a) dargestellte Problem nicht erkannt hat, d.h. sich für ihn das Problem (und damit die erfindungsgemäße Lösung des unerkannten Problems) nicht stellte. Zum zweiten nicht, weil selbst wenn er das Problem der fehlenden Röntgenopazität bei Infiltranten erkannt hätte, er keine Lösung auf dem Gebiet der herkömmlichen Restaurationsmaterialien gefunden hätte ohne erfinderisch tätig zu werden. Denn der Fachmann musste aufgrund seines Fachwissens davon ausgehen, dass nanoskalige Füllstoffe ungünstigerweise stark die Viskosität des Infiltrants erhöhen würden. Diese Annahme basiert insbesondere auf der für flüssige Dentalkomposite beobachteten Eigenschaft, das bei Zugabe von nanoskaligen Füllstoffen die Viskosität stark ansteigt.

Ein Anstieg der Viskosität würde der Fachmann bei der Verwendung von nanoskaligen Füllstoffen in Infiltranten jedoch gerade nicht wünschen oder in Kauf nehmen, da er bei der zu erwartenden höheren Viskosität nicht mehr von einer ausreichenden Penetration des Infiltranten in die Zahnschmelzläsion ausgehen kann. Mit anderen Worten, bei den bekannten nanoskaligen Füllstoffen erwartet der Fachmann deshalb, dass sie den Penetrationskoeffizienten eines Infiltranten so stark herabsetzen, dass dessen Penetrationsfähigkeit verloren geht oder stark verschlechtert wird.

Vorliegende Erfindung hat überraschenderweise festgestellt, dass sich entgegen dieser Annahme Infiltranten mit nanoskaligen Füllstoffen zur bestimmungsgemäßen Verwendung als Infiltrant für Dentalapplikationen bereitstellen lassen.

Wie die Versuchsbeispiele vorliegender Erfindung belegen, löst vorliegende Erfindung die technische Aufgabe vorliegender Erfindung tatsächlich. Die Versuche belegen, dass sich bei den erfindungsgemäßen Infiltranten ausreichend hohe Penetrationskoeffizienten mit einer hohen Röntgenopazität kombinieren lassen.

Zunächst seien einige im Rahmen der Erfindung verwendete Begriffe erläutert.

Der Begriff Infiltrant bezeichnet eine Flüssigkeit, die als ungehärtetes Harz in eine Zahnschmelzläsion (einen porösen Festkörper) eindringen kann. Nach dem Eindringen kann der Infiltrant dort ausgehärtet werden.

Das Eindringen einer Flüssigkeit (ungehärtetes Harz) in einen porösen Festkörper (Zahnschmelzläsion) wird durch die Washburn-Gleichung (Gleichung 1, siehe unten) physikalisch beschrieben. Bei dieser Gleichung wird angenommen, dass der poröse Festkörper ein Bündel offener Kapillaren darstellt (Buckton G., Interfacial phenomena in drug delivery and targeting. Chur, 1995); in diesem Fall wird das Eindringen der Flüssigkeit von Kapillarkräften getrieben.

$$d^2 = \left( \frac{\gamma \cdot \cos\theta}{2\eta} \right) r \cdot t \qquad \text{- Gleichung 1 -}$$

d    Abstand, um den sich das flüssige Harz bewegt

$\gamma$    Oberflächenspannung des flüssigen Harzes (gegen Luft)

$\theta$    Kontaktwinkel des flüssigen Harzes (gegen Zahnschmelz)

$\eta$    dynamische Viskosität des flüssigen Harzes

r    Kapillarradius (Porenradius)

t    Eindringdauer

Der in Klammern stehende Ausdruck der Washburn-Gleichung wird als Penetrationskoeffizient (PK, Gleichung 2, siehe unten) bezeichnet (Fan P. L. et al., Penetrativity of sealants. J. Dent. Res., 1975, 54: 262-264). Der PK setzt sich aus der Oberflächenspannung der Flüssigkeit gegen Luft ($\gamma$), dem Cosinus des Kontaktwinkels der Flüssigkeit gegen Zahnschmelz ($\theta$) und der dynamischen Viskosität der Flüssigkeit ($\eta$) zusammen. Je größer der Wert des Koeffizienten ist, um so schneller dringt die Flüssigkeit in eine gegebene Kapillare oder ein gegebenes poröses Bett ein. Dies bedeutet, dass ein hoher Wert des PK durch hohe Oberflächenspannungen, niedrige Viskositäten und niedrige Kontaktwinkel erzielt werden kann, wobei der Einfluss des Kontaktwinkels vergleichsweise gering ist.

$$PK = \left( \frac{\gamma \cdot \cos\theta}{2\eta} \right) \qquad \text{- Gleichung 2 -}$$

PK    Penetrationskoeffizient

γ    Oberflächenspannung des flüssigen Harzes (gegen Luft)

θ    Kontaktwinkel des flüssigen Harzes (gegen Zahnschmelz)

η    dynamische Viskosität des flüssigen Harzes

Überraschenderweise wurde gefunden, dass Infiltranten enthaltend nanoskalige Füllstoffe Penetrationskoeffizienten über 50, bevorzugt über 100 cm/s aufweisen können.

Bei den erfindungsgemäß geeigneten eingesetzten nanoskaligen Füllstoffen (i) handelt es sich um Metall-, Halbmetall- oder Mischmetalloxide, -silikate, -nitride, -sulfate, - titanate, -zirkonate, -stannate, -wolframate, -phosphate, halogenide oder um eine Mischung aus diesen Verbindungen. Zur Gruppe der Halbmetalle, deren Eigenschaften (vor allem Aussehen und elektrische Leitfähigkeit) zwischen denen der Metalle und der Nichtmetalle liegen, gehören Bor, Silizium, Germanium, Arsen, Antimon, Bismut, Selen, Tellur und Polonium (vgl. Römpp Chemie Lexikon, Georg Thieme Verlag, 1990, S. 1711). Die Gruppe der Metalle ist im Periodensystem links von der Gruppe der Halbmetalle zu finden, d.h. dazu gehören die Hauptgruppenmetalle, Nebengruppenmetalle, Lanthanide und Actinide. Unter dem Begriff Mischmetalloxid, - nitrid, usw. ist hier eine chemische Verbindung zu verstehen, in der mindestens zwei Metalle und/oder Halbmetalle zusammen mit dem entsprechenden (Nicht-)metallanion (Oxid, Nitrid, usw. ) chemisch miteinander verbunden sind.

Bei den erfindungsgemäß einsetzbaren nanoskaligen Füllstoffen handelt es sich bevorzugt um Aluminiumoxid, Zirkondioxid, Titandioxid, Zinkoxid, Zinndioxid, Ceroxid, Aluminium-Silizium-Oxide, Silizium-Zink-Oxide, Silizium-Zirkon-Oxide, Eisenoxide und deren Mischungen mit Siliziumdioxid, Indiumoxide und deren Mischungen mit Siliziumdioxid und/oder Zinndioxid, Siliziumdioxid, Bornitrid, Strontiumsulfat, Bariumsulfat, Strontiumtitanat, Bariumtitanat, Natriumzirkonat, Kaliumzirkonat, Magnesiumzirkonat, Calciumzirkonat, Strontiumzirkonat, Bariumzirkonat, Natriumwolframat, Kaliumwolframat, Magnesiumwolframat, Calciumwolframat, Strontiumwolframat und/oder Bariumwolframat.

Bevorzugt eingesetzte nanoskalige röntgenopake Füllstoffe sind ausgewählt aus der Gruppe bestehend aus Salzen der Seltenerdmetalle, des Scandiums, des Yttriums, des Bariums und Strontiums, oder Wolframaten. Als schwerlösliche Salze eigenen sich vorzugsweise Sulfate, Phosphate oder Fluoride.

Unter den Salzen der Seltenerdmetalle (Elemente 57-71), des Scandiums oder des Yttriums sind die Trifluoride bevorzugt. Zu den bevorzugten Seltenerdmetallen zählen Lanthan, Cer, Samarium, Gadolinium, Dysprosium, Erbium oder Ytterbium. Unter deren Salzen sind die Fluoride bevorzugt, insbesondere Ytterbiumtrifluorid (YbF3). Bevorzugte Barium- und Strontiumsalze sind Fluoride, Phosphate und Sulfate, insbesondere die Sulfate.

Der Ausdruck "Wolframat" umfasst Metallverbindungen der Orthowolframate und Polywolframate, wobei erstere bevorzugt sind.

Das Metallwolframat ist vorzugsweise eine Wolframatverbindung eines mehrwertigen Metalls, insbesondere eines zwei- oder dreiwertigen Metalls. Geeignete zweiwertige Metalle umfassen Erdalkalimetalle, wie Magnesium, Calcium, Strontium oder Barium, insbesondere Calcium, Strontium oder Barium. Strontium- und Bariumwolframate zeichnen sich durch eine besonders hohe Röntgenopazität aus, da in diesen Verbindungen zwei gute Kontrastbildner miteinander kombiniert werden. Bevorzugte dreiwertige Metalle umfassen Scandium, Yttrium oder Seltenerdmetalle, wie Lanthan, Cer, Samarium, Gadolinium, Dysprosium, Erbium oder Ytterbium. Auch hier ergibt sich eine besonders hohe Röntgenopazität dadurch, dass ein guter Kontrastbildner (Wolframat) mit einem stark kontrastbildenden Metall kombiniert wird.

Darüber hinaus ist es möglich, die erfindungsgemäß eingesetzten Wolframate (bzw. auch die anderen nanoskaligen Salze) mit Metallatomen zu dotieren. Vorzugsweise wird hierzu das Wirtsgittermetall durch den Dotierungsstoff in einer Menge von bis zu 50 Mol-%, stärker bevorzugt 0,1 bis 40 Mol-%, noch stärker bevorzugt 0, 5 bis 30 Mol-%, insbesondere 1 bis 25 Mol-% ersetzt. Der gewählte Dotierungsstoff kann zur Röntgenopazität beitragen. Aus analytischen Gründen kann es jedoch auch von Interesse sein, eine oder mehrere dotierende Metalle zu wählen, die lumineszierende Eigenschaften, insbesondere Photolumineszenz vermitteln. Zu diesem Zweck geeignete Dotierungsstoffe sind fachbekannt und werden oft unter einem vom Wirtsgittermetall verschiedenen Lanthanid ausgewählt. Beispiele umfassen die kombinierte Dotierung mit Eu und Bi, oder die Dotierung von Ce in Kombination mit Nd, Dy oder Tb, oder Er in Kombination mit Yb. Gleichermaßen kann man ein Wolframat als Wirtsgitter mit einem geeigneten Lanthanidion oder einem anderen Metallion, z. B. $Bi^{3+}$ oder $Ag^+$ dotieren.

Die erfindungsgemäßen nanoskaligen röntgenopaken Füllstoffe weisen vorzugsweise folgende mittlere Primärpartikelgrößen $d_{50}$ bzw. Bereiche dieser Partikelgrößen auf:

- kleiner 1000 nm, kleiner 700 nm, kleiner 500 nm, kleiner 200 nm, kleiner 100 nm, kleiner 25 nm,
- zwischen 1 nm und 80 nm, zwischen 4 nm und 60 nm, zwischen 6 nm und 50 nm, zwischen 0,5 nm und 22 nm, zwischen 1 nm und 20 nm, zwischen 1 nm und 10 nm oder zwischen 1 nm und 5 nm.

Besonders bevorzugt sind vereinzelt vorliegende, nicht aggregierte und nicht agglomerierte nanosklalige Füllstoffe. Weiterhin bevorzugt sind Füllstoffe mit unimodaler Teilchengrößenverteilung.

Der erfindungsgemäße nanoskalige Füllstoff weist eine BET-Oberfläche (gemäss DIN 66131 bzw. DIN ISO 9277) zwischen 15 m2/g und 600 m2/g, bevorzugt zwischen 30 m2/g und 500 m2/g und besonders bevorzugt zwischen 50 m2/g und 400 m2/g auf.

Geeignete Gehalte des nanoskaligen röntgenopaken Füllstoffs im Infiltranten, bezogen auf die Gesamtmasse des Infiltranten mit allen Inhaltsstoffen, liegen bei 1 bis 30 Gew.-%, bevorzugt bei 5 bis 25 Gew.-%, besonders bevorzugt bei 10 bis 20 Gew.-%, weiterhin bevorzugte Bereiche sind 1 bis 5 Gew.-%, 5 bis 10 Gew.-%, 10 bis 15 Gew.-% und 15 bis 20 Gew.-%.

Der erfindungsgemäße Infiltrant kann weiterhin nanoskalige Füllstoffe mit einem remineralisierenden Effekt enthalten. Bevorzugt sind Metallfluoride oder (Fluor)(Chlor)Hydroxylapatite oder auch Fluoraluminiumsilikate.

Der nanoskalige Füllstoff kann organisch modifiziert sein.

Bei dieser organischen Modifikation werden funktionelle Gruppen auf die Oberfläche der Nanopartikel aufgebracht, die zum einen entweder kovalent oder adsorptiv an die Nanopartikel gebunden sind und die zum anderen mit der Monomermatrix (organische Bindemittel) chemisch reagieren können oder eine hohe Affinität zu dem organischen Bindemittel haben.

Die organische Modifikation der Oberfläche der Nanofüllstoffe erfolgt bevorzugt durch Behandeln mit einem Siloxan, Chlorsilan, Silazan, Titanat, Zirkonat, Wolframat oder mit einer organischen Säure, wie bspw. organische Phosphon-, oder Phosphorsäure, oder organische Säuren wie sie z. B. in US 6,387, 981 beschrieben sind, einem organischen Säurechlorid oder -anhydrid. Die Siloxane, Chlorsilane, Silazane, Titanate, Zirkonate und Wolframate haben besonders bevorzugt die allgemeinen Formeln

- $Si(OR')_nR_{4-n}$,
- $SiCl_nR_{4-n}$,
- $(R_mR''_{3-m}Si)_2NH$,
- $Ti(OR')_nR_{4-n}$,
- $Zr(OR')_nR_{4-n}$ und
- $W(OR')_nR_{6-n}$,

wobei

- m = 1, 2 oder 3 ist,

- n = 1, 2 oder 3, vorzugsweise n gleich 3 ist,

- die über den Sauerstoff gebundene Gruppe R' und die Gruppe R" eine beliebige organische funktionelle Gruppe, vorzugsweise eine Alkylgruppe, besonders bevorzugt eine Methyl-, Ethyl-, Propyl- oder Isopropylgruppe ist und

- die funktionelle Gruppe R eine beliebige organische Gruppe und direkt über ein Kohlenstoffatom an das Silizium, Titan, Zirkon oder Wolfram gebunden ist.

Wenn m oder n 1 oder 2 ist, können die Gruppen R gleich oder verschieden sein. R wird bevorzugt so ausgewählt, dass es eine oder mehrere funktionelle Gruppen besitzt, die mit Monomeren chemisch reagieren können oder eine hohe Affinität zur Monomermatrix haben. Diese funktionellen Gruppen sind auch in den ebenfalls zur organischen Oberflächenmodifikation einsetzbaren, oben aufgeführten organischen Säuren, Säurechloriden und -anhydriden enthalten. Es handelt sich bevorzugt um Acrylat-, Methacrylat-, Cyanacrylat-, Acrylamid-, Methacrylamid-, Vinyl-, Allyl-, Styryl-, Epoxid-, Oxetan-, Vinylether-, Amino-, Säure-, Säureester-, Säurechlorid-, Phosphat-, Phosphonat-, Phosphit-, Thiol-, Alkohol- und/oder Isocyanatgruppen.

Der erfindungsgemäße Infiltrant kann weiterhin dem Fachmann bekannte röntgenopake organische Verbindungen enthalten. Geeignete röntgenopake organische Verbindungen sind metallorganische Verbindungen, aliphatische, cyclische oder aromatische Halogenide, insbesondere Bromverbindungen, Schwermetallionen enthaltende Monomere sowie halogenhaltige Monomere. Bevorzugte erfindungsgemäße röntgenopake organische Verbindungen sind (meth)acrylierte Triphenylbismutderivate sowie iodsubstituierte Benzoesäureester und -amide.

Geeignete im erfindungsgemäßen Infiltranten enthaltene Monomere sind ausgewählt aus vernetzenden Monomere mit zwei polymerisierbaren Gruppen, bevorzugt sind Ester der Acryl- und/oder Methacrylsäure. Sie können vorzugsweise ausgewählt sein aus der Gruppe bestehend aus DDDMA, 1,10-Decandioldimethacrylat; PEG400DA, Polyethylenglycol 400 Diacrylat; PEG400DMA, Polyethylenglycol 400 Dimethacrylat; PEG300DA, Polyethylenglycol 300 Diacrylat; PEG300DMA, Polyethylenglycol 300 Dimethacrylat; BPA(EO)10DMA, Ethoxyliertes (10) Bisphenol-A-Dimethacrylat; BPA(EO)30DMA, Ethoxyliertes (30) Bisphenol-A-Dimethacrylat; PEG200DA, Polyethylenglycol 200 Diacrylat; PEG600DA, Polyethylenglycol 600 Diacrylat; NPG(PO)2DA Propoxyliertes (2) Neopentylglycol Diacrylat; BPA(EO)2DA, Ethoxyliertes (4) Bisphenol-A-Diacrylat; BPA(PO)2DMA, Propoxyliertes (2) Bisphenol-A-Dimethacrylat; Bis-GMA, 2,2-bis[4-(2-Hydroxy-3-Methacryloxypropoxy)phenyl]propan; UDMA, 1,6-bis(Methacryloxy-2-Ethoxycarbonylamino)-2,4,4-trimethylhexan; EGDMA, Ethylenglycoldimethacrylat; TEDMA, Triethylenglycoldimethacrylat; 4EGDMA, Tetraethylenglycoldimethacrylat; BDMA, 1,3-Butylenglycoldimethacrylat; HDDMA, 1,6-Hexandioldimethacrylat; 1,4-Butylendioldiacrylat; 4EDA, Tetraethylenglycoldiacrylat; NDDA, 1,9- Nonandioldiacrylat; DEGDMA, Diethylenglycoldimethacrylat; PDDMA, 1,5-Pentandioldimethacrylat; BDDMA, 1,4-Butandioldimethacrylat; PRDMA, 1,3-Propandioldimethacrylat; und Dimethylol tricyclo[5.2.1.0]decandimethacrylat.

Weiterhin geeignete im erfindungsgemäßen Infiltranten enthaltene Monomere sind ausgewählt aus vernetzenden Monomere mit mindestens drei polymerisierbaren Gruppen der nachfolgenden Formel

$$R^1[X_k\ R^2_l\ Y_m]_n$$

mit folgenden Bedeutungen

$R^1$ ist ein linearer oder verzweigter Kohlenwasserstoff mit 3-24 C-Atomen, enthaltend Alkyl, Cycloalkyl oder Aryl;

optional enthaltend O, N, Si, S, P als Heteroatome, beispielsweise Siloxan und/oder Cyclosiloxan und/oder Carbosilan und/oder Cyclocarbosilan und/oder insbesondere Ether- oder Polyethergruppen, Polyestergruppen, Polysiloxangruppen oder Polycarbosilangruppen;

optional substituiert durch Hydroxyl und/oder Carbonyl und/oder Halogen (bevorzugt Fluor) und/oder Ammoniumalkylengruppen und/oder Siloxan und/oder Cyclosiloxan und/oder Carbosilan und/oder Cyclocarbosilan und/oder Säure- oder Säurederivatgruppen, insbesondere Carbonsäuregruppen, Phosphorsäuregruppen, Phosphonsäuregruppen oder Sulfonsäuregruppen;

$R^2$ ist ein linearer oder verzweigter Kohlenwasserstoff mit 1-16 C-Atomen, enthaltend Alkyl, Cycloalkyl oder Aryl;

optional enthaltend O, N, Si, S, P als Heteroatome, beispielsweise Siloxan und/oder Cyclosiloxan und/oder Carbosilan und/oder Cyclocarbosilan und/oder insbesondere Ether- oder Polyethergruppen, Polyestergruppen, Polysiloxangruppen oder Polycarbosilangruppen;

optional substituiert durch Hydroxyl und/oder Carbonyl und/oder Halogen (bevorzugt Fluor) und/oder Ammoniumalkylengruppen und/oder Siloxan und/oder Cyclosiloxan und/oder Carbosilan und/oder Cyclocarbosilan und/oder Säure- oder Säurederivatgruppen, insbesondere Carbonsäuregruppen, Phosphorsäuregruppen, Phosphonsäuregruppen oder Sulfonsäuregruppen;

X ist eine verknüpfende Gruppe gleich oder verschieden ausgewählt aus einer Ethergruppe, Carbonylgruppe, Estergruppe, Amidgruppe, Urethangruppe oder Harnstoffgruppe;

Y ist eine Gruppe gleich oder verschieden enthaltend eine polymerisierbare Doppelbindung und/oder eine ringöffnend polymerisierbare Gruppe und/oder eine Thiolgruppe; bevorzugt Vinyl, (Meth)acrylat, (Meth)acrylamid oder Epoxidgruppen;

k ist 0 oder 1;

l ist 0 oder 1

m ist mindestens 1;

n ist mindestens 1

n x m ist mindestens 3.

Geeignete niedrigviskose Monomeren mit mindestens drei polymerisierbaren Gruppen sind beispielsweise TMPTMA, Trimethylolpropantrimethacrylat, TMPTA, Trimethylolpropantri(meth)acrylat; DTMPTA; Di-Trimethylolpropantetra-(meth)acrylat; DiPENTA, Di-Pentaerythritolpenta(meth)-acrylat; oder DPEHA, Di-Pentaerythritolhexa(meth)acrylat.

Weiterhin geeignete niedrigviskose Monomere mit mindestens drei polymerisierbaren Gruppen basieren bspw. auf alkoxylierten Mehrfachalkoholen (Tri, Tetra-, Penta, Hexa-, Polyole)wie Trimethylolpropan, Ditrimethylolpropan, Glycerol, Pentaerythritol oder Dipentaerythritol.

Besonders geeignet sind dabei (Meth)acrylester von alkoxylierten Mehrfachalkoholen wie beispielsweise ethoxyliertes Trimethylolpropan-trimethacrylat, ethoxyliertes Trimethylolpropan-triacrylat, propoxyliertes Trimethylolpropan-trimethacrylat, propoxyliertes Trimethylolpropantriacrylat, ethoxyliertes Pentaerythritol-trimethacrylat, ethoxyliertes Pentaerythritol-triacrylat, ethoxyliertes Pentaerythritol-tetramethacrylat, ethoxyliertes Pentaerythritol-tetraacrylat, ethoxyliertes Di-Pentaerythritol-trimethacrylat, ethoxyliertes Di-Pentaerythritol-tetramethacrylat, ethoxyliertes Di-Pentaerythritolpentamethacrylat, ethoxyliertes Di-Pentaerythritolhexamethacrylat, ethoxyliertes Di-Pentaerythritol-triacrylat, ethoxyliertes Di-Pentaerythritol-tetraacrylat, ethoxyliertes Di-Pentaerythritol-pentaacrylat, ethoxyliertes Di-Pentaerythritol-hexaacrylat, propoxyliertes Pentaerythritol-trimethacrylat, propoxyliertes Pentaerythritol-triacrylat, propoxyliertes Pentaerythritol-tetramethacrylat, propoxyliertes Pentaerythritol-tetraacrylat, propxyliertes Di-Pentaerythritol-trimethacrylat, propoxyliertes Di-Pentaerythritol-tetramethacrylat, propoxyliertes Di-Pentaerythritolpentamethacrylat, propoxyliertes Di-Pentaerythritolhexamethacrylat, propoxyliertes Di-Pentaerythritol-triacrylat, propoxyliertes Di-Pentaerythritol-tetraacrylat, propxyliertes Di-Pentaerythritol-pentaacrylat, propoxyliertes Di-Pentaerythritol-hexaacrylat.

An Alkohole angebunde Alkoxygruppen stellen (Molekül)Kettenverlängerer dar. Die Kettenverlängerung kann vorzugsweise durch Ethoxylierung oder Propoxylierung erreicht werden. Für die Kettenverlängerung kommen weitere Verknüpfungsmöglichkeiten bspw. Etherbindungen, Esterbindungen, Amidbindungen, Urethanbindungen u.ä. in Frage, an die sich bevorzugt wiederum Ethylenglykol- oder Propylenglykolgruppen anschließen können.

Die kettenverlängernde Gruppe ist bevorzugt endständig mit den vernetzenden Gruppen, bevorzugt einer Methacrylat, einer Acrylatgruppe, Methacrylamid oder Acrylamidgruppe funktionalisiert.

Als Vernetzungspunkt wird die Position der vernetzenden polymerisierbaren Gruppe bspw. die Position einer C=C-Doppelbindung im Monomer angesehen.

Die Kettenlänge ist bevorzugt derart, dass der Abstand der Vernetzungspunkte mindestens 7, bevorzugt mindestens 9, weiter bevorzugt 10 bis 30, besonders bevorzugt 11 bis 21 Bindungslängen beträgt. Der Abstand beträgt bevorzugt weniger als 50 Bindungslängen.

Mit Abstand der Vernetzungspunkte, ist der kürzeste Abstand der vernetzenden Gruppen bspw. zweier C=C-Doppelbindungen entlang des Moleküls zu verstehen. Es ist also nur die Konstitution des Moleküls gemeint, nicht etwa die wirkliche räumliche Lage der Gruppen zueinander, etwa durch Konfiguration oder Konformation bedingt.

Unter Bindungslänge ist der Abstand zweier Atome im Molekül zu verstehen, egal welche Art kovalente Bindung vorliegt und welche exakte Bindungslänge die einzelne kovalente Bindung aufweist.

Der bevorzugte Anteil dieser Monomeren hängt von der Anzahl der vernetzenden Gruppen in der Monomermischung, der Größe der kettenverlängernden Gruppen und dem resultierenden PK ab.

Der erfindungsgemäße Infiltrant kann zusätzlich Monomere mit einer polymerisierbaren Gruppe enthalten. Diese können bevorzugt ausgewählt sein aus der Gruppe bestehend aus MMA, Methylmethacrylat; EMA, Ethylmethacrylat; n-BMA, n-Butylmethacrylat; IBMA, Isobutylmethacrylat, t-BMA, tert-Butylmethacrylat; EHMA, 2-Ethylhexylmethacrylat; LMA, Laurylmethacrylat; TDMA, Tridecylmethacrylat; SMA, Stearylmethacrylat; CHMA, Cyclohexylmethacrylat; BZMA, Benzylmethacrylat; IBXMA, Isobornylmethacrylate; MAA, Methacrylsäure; HEMA, 2-Hydroxyethylmethacrylat; HPMA, 2-Hydroxypropylmethacrylat; DMMA, Dimethylaminoethylmethacrylat; DEMA, Diethylaminoethylmethacrylat; GMA, Glycidylmethacrylat; THFMA, Tetrahydrofurfurylmethacrylat; AMA, Allylmethacrylat; ETMA, Ethoxyethylmethacrylat; 3FM, Trifluorethylmethacrylat; 8FM, Octafluorpentylmethacrylat; AIB, Isobutylacrylat; TBA, tert-Butylacrylat; LA, Laurylacrylat; CEA, Cetylacrylat; STA, Stearylacrylat; CHA, Cyclohexylacrylat; BZA, Benzylacrylat; IBXA, Isobornylacrylat; 2-MTA, 2-Methoxyethylacrylat; ETA, 2-Ethoxyethylacrylat; EETA, E-thoxyethoxyethylacrylat; PEA, 2-Phenoxyethylacrylat; THFA, Tetrahydrofurfurylacrylat; HEA, 2-Hydroxyethylacrylat; HPA, 2-Hydroxypropylacrylat; 4HBA, 4-Hydroxybutylacrylat; DMA, Dimethylaminoethylacrylat; 3F, Trifluorethylacrylat; 17F, Heptadecafluorodecylacrylat; 2-PEA, 2-Phenoxyethylacrylat; TBCH, 4-tert-butylcyclohexylacrylat; DCPA, Dihydrodicyclopentadienylacrylat; EHA, 2-Ethylhexylacrylat; und 3EGMA, Triethylenglycolmonomethacrylat.

Es kann von Vorteil sein, wenn die im Infiltranten enthaltenen Monomeren zusätzliche funktionelle Gruppen aufweisen wie Säuregruppen, insbesondere Carbonsäuregruppen, Phosphorsäuregruppen, Phosphonsäuregruppen oder Sulfonsäuregruppen oder Ammoniumalkylengruppen oder Halogen, insbesondere fluoriertes Alkylen.

Die Monomere, Monomermischungen und/oder Infiltranten haben bevorzugt eine Dynamische Viskosität kleiner 50 mPas, weiter bevorzugt kleiner 30 mPas, besonders bevorzugt kleiner 15 mPas.

Der Anteil vernetzender Monomere mit mindestens drei polymerisierbaren Gruppen kann zwischen 0 und 50 Gew.-%, weiter zwischen 10 und 30 Gew.-% liegen. Der Anteil vernetzender Monomere mit zwei polymerisierbaren Gruppen liegt bei einer bevorzugten Ausführungsform zwischen 100 und 50 Gew.-%. Ein weiterer bevorzugter Bereich liegt zwischen 90 und 70 Gew.-%.

Die erfindungsgemäßen Infiltranten lassen sich je nach chemischem Aufbau der enthaltenen Monomere radikalisch, anionisch oder kationisch erhärten. Bevorzugt sind die Monomere radikalisch oder kationisch härtbar.

Die radikalische Härtung der erfindungsgemäßen Monomere kann durch Vinylpolymerisation geeigneter Doppelbindungen erfolgen. Besonders geeignet sind hier (Meth)acrylate, (Meth)acrylamide, Styrylverbindungen, Cyanacrylate und Verbindungen mit ähnlich gut radikalisch polymerisierbaren Doppelbindungen. Eine weitere Möglichkeit der radikalischen Härtung besteht in der ringöffnenden Polymerisation von cyclischen Vinylverbindungen wie den in EP 1 413 569, EP 1 741 419 und EP 1 688 125 beschriebenen Vinylcyclopropanen oder anderen cyclischen Systemen wie Vinylidensubstituierten Orthospirocarbonaten oder Orthospiroestern. Eine weitere Möglichkeit besteht auch in der Copolymerisation der ringöffnend polymerisierenden Systeme mit den oben genannten einfach polymerisierenden Doppelbindungen.

Die radikalische Härtung kann darüber hinaus durch eine unter dem Begriff Thiol-En-Reaktion bekannte Stufenreaktionen, wie in WO 2005/086911 beschrieben, erfolgen.

Die kationische Härtung der erfindungsgemäßen Monomere kann ebenfalls durch ringöffnende wie auch durch Vinylpolymerisation erfolgen. Geeignete Vinylpolymere sind Vinylether, Styrylverbindungen und weitere Verbindungen mit elektronenreichen Vinylgruppen. Geeignete ringöffnend polymerisierende Monomere sind Verbindungen, die Epoxid-, Oxetan-, Aziridin-, Oxazolin oder Dioxolangruppen tragen. Weitere ringöffnend polymerisierende Gruppen können der Literatur; bspw.: K.J. Ivin, T. Saegusa, (Eds.), Vol.2, Elsevier Appl. Sci. Publ., London 1984; entnommen werden. Besonders geeignet sind siliziumhaltige Epoxidmonomere, wie sie in WO 02/066535 oder WO 2005/121200 beschrieben sind. Besonders vorteilhaft bei der Verwendung von Epoxiden oder Oxetanen ist der geringe Polymerisationsschrumpf wie auch die geringe Inhibitionsschicht dieser Materialien.

Die Mischung unterschiedlicher Monomeren dient ebenfalls der Abstimmung der mechanischen Eigenschaften wie Härte und Festigkeit, der Durchhärtetiefe bzw. des Polymerisationsgrades, des Restmonomergehaltes, der Schmierschichtausprägung, des Schrumpfs, der Stabilität, der Wasseraufnahme sowie insbesondere der Spannungsfreiheit unter Erhalt einer hohen Penetrationsfähigkeit (PK > 50).

Insbesondere ist auch die Wasserverträglichkeit der Monomeren von Bedeutung etwa für den Fall, dass die Schmelzläsion nach der Vorbereitung (Ätzen, Spülen, Trocknen) noch Restfeuchtigkeit aufweist. Bestimmte Monomere können Restfeuchtigkeit aufnehmen und so die Penetration weiter verbessern. Hierfür eignen sich besonders wasserlösliche und/oder phasenvermittelnde Ester der (Meth)acrylsäure, bspw. HEMA, 2-Hydroxyethylmethacrylat oder GDMA, Glycerindimethacrylat oder GMA, Glycerinmonomethacrylat.

Die Mischung mit weiteren Monomeren kann weiterhin insbesondere der Abstimmung weiterer vorteilhafter Eigenschaften wie hohe Oberflächenglätte (plaqueverhindernd), Fluoridfreisetzung, Röntgenopazität, Haftung am Schmelz, Langzeitfarbstabilität, Biokompatibilität u.s.w. dienen.

Der Infiltrant kann auch dem Fachmann bspw. aus WO 02/062901, WO 2006/031972 oder EP 1 714 633 bekannte und im Dentalbereich gebräuchliche hyperverzweigte Monomere, bspw. Dendrimere aufweisen, insbesondere um den Restmonomergehalt zu senken und die Biokompatibilität zu verbessern.

Der Infiltrant kann dem Fachmann bspw. aus EP 1 285 947 oder EP 1 849 450 bekannte und im Dentalbereich gebräuchliche bakterizide Monomere enthalten.

Die Monomermischungen haben dabei einen PK > 50, bevorzugt > 100, besonders bevorzugt > 200.

Der Infiltrant enthält Mittel zur Härtung des Infiltranten. Die Mittel zur Härtung können dem Fachmann bekannte und im Dentalbereich gebräuchliche Initiatoren insbesondere lichtaktivierte Initiatörsysteme aber auch chemisch aktivierende Initiatoren sein oder Mischungen der verschiedenen Systeme.

Die hier verwendbaren Initiatoren können z. B. Photoinitiatoren sein. Diese sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm und optional durch diezusätzliche Reaktion mit einem oder mehreren Coinitiatoren die Aushärtung des Materials bewirken können. Bevorzugt werden hier Phosphinoxide, Bezoinether, Benzilketale, Acetophenone, Benzophenone, Thioxanthone, Bisimidazole, Metallocene, Fluorone, a-Dicarbonylverbindungen, Aryldiazoniumsalze, Arylsulfoniumsalze, Aryliodoniumsalze, Ferroceniumsalze, Phenylphosphoniumsalze oder eine Mischung aus diesenVerbindungen eingesetzt.

Besonders bevorzugt werden Diphenyl-2,4,6-trimethylbenzoylphosphinoxid, Benzoin, Benzoinalkylether, Benzildialkylketale, a-Hydroxy-acetophenon, Dialkoxyacetophenone, a-Aminoacetophenone, i-Propylthioxanthon, Campherchinon,

Phenylpropandion, 5,7-Diiodo-3-butoxy-6-fluoron, (eta-6-10 Cumol)(eta-5-cyclopentadienyl)eisen-hexafluorophosphat, (e-ta-6-Cumol)(eta-5-cyclopentadienyl)eisen-tetrafluoroborat, (eta-6-Cumol)(eta-5-cyclopentadienyl)eisenhexafluoroantimonat, substituierte Diaryliodoniumsalze, Triarylsulfoniumsalze oder eine Mischung aus diesen Verbindungen eingesetzt.

Als Coinitiatoren für eine photochemische Aushärtung werden bevorzugt tertiäre Amine, Borate, organische Phosphite, Diaryliodoniumverbindungen, Thioxanthone, Xanthene, Fluorene, Fluorone, a-Dicarbonylverbindungen, kondensierte Polyaromaten oder eine Mischung aus diesen Verbindungen eingesetzt.

Besonders bevorzugt werden N,N-Dimethyl-ptoluolidin, N,N-Dialkyl-alkyl-aniline, N,N-Dihydroxyethylp-toluidin, 2-Ethylhexyl-p-(dimethyl-amino)-benzoat, Butyrylcholintriphenylbutyl-borat oder eine Mischung aus diesen Verbindungen eingesetzt.

Der Infiltrant kann aus einem Kit mit wenigstens zwei Komponenten hergestellt werden. Zwei Komponenten enthaltende Infiltranten haben den Vorteil, dass sie selbsthärtend (chemische Aushärtung) gestaltet werden können. In einer Ausführungsform enthält eine erste Komponente Monomere und chemisch aktivierbare Initiatoren und eine zweite Komponente geeignete Aktivatoren.

Für eine chemische Aushärtung bei Raumtemperatur wird i. a. ein Redoxinitiatorsystem verwendet, das aus einem bzw. mehreren Initiatoren und einem als Aktivator dienenden Coinitiator bzw. Coinitiatoren besteht. Aus Gründen der Lagerstabilität werden Initiator bzw. Initiatoren und Coinitiator bzw. Coinitiatoren in räumlich voneinander getrennten Teilen des erfindungsgemäßen Infiltranten eingearbeitet, d. h. es liegt ein mehrkomponentiges, bevorzugt ein zweikomponentiges Material vor. Als Initiator bzw. Initiatoren werden bevorzugt anorganische und/oder organische Peroxide, anorganische und/oder organische Hydroperoxide, Barbitursäurederivate, Malonylsulfamide, Protonensäuren, Lewis- oder Broensted-Säuren bzw. Verbindungen, die solche Säuren freisetzen, Carbeniumionen-Donatoren wie z. B. Methyltriflat oder Triethylperchlorat oder eine Mischung aus diesen Verbindungen und als Coinitiator bzw. als Coinitiatoren bevorzugt tertiäre Amine, Schwermetallverbindungen, insbesondere Verbindungen der 8. und der 9. Gruppe des Periodensystems ("Eisen- und Kupfergruppe"), Verbindungen mit ionogen gebundenen Halogenen oder Pseudohalogenen wie z. B. quartäre Ammoniumhalogenide, schwache Broenstedt-Säuren wie z. B. Alkohole und Wasser oder eine Mischung aus diesen Verbindungen eingesetzt.

In einer besonders einfachen Ausführungsform ist das Gerät zum Auftragen des Infiltranten (Applikationshilfe) auf den Zahn mit dem Aktivator getränkt oder belegt.

In einer weiteren Ausführungsform enthält eine erste Komponente Monomere und als Initiatoren Salze CH-azider Verbindungen wie Barbitursäurederivate und eine zweite Komponente Monomere und eine aktivierende Komponente, bevorzugt eine stärker azide Säure als die CH-azide Verbindung.

In einer besonders einfachen Ausführungsform enthält ein Gerät zum Auftragen des Infiltranten (Applikationshilfe) auf den Zahn eine Mischkammer und/oder Mischelemente enthaltende Kanülen.

Der Infiltrant kann Stabilisatoren enthalten. Bevorzugt werden UV-Stabilisatoren. Geeignete UV-Stabilisatoren sind dem Fachmann bekannt beispielhaft sei hier Chimasorb® und Tinuvin® (Ciba) genannt.

Der Infiltrant kann Lösungsmittel enthalten. Bevorzugt sind flüchtige Lösungsmittel wie bspw. Alkohole, Ketone, Ether, u.s.w.

Bevorzugt enthält der Infiltrant weniger als ungefähr 20 Masse-%, weiter bevorzugt weniger als ungefähr 10 Masse-%, besonders bevorzugt kein Lösungsmittel.

Der Infiltrant kann mindestens einen fluoreszierenden Farbstoff und/oder Farbpigmente enthalten, um das Erscheinungsbild zu verbessern bzw. dem Zahnschmelz anzupassen. Geeignete fluoreszierende Farbmittel sind dem Fachmann bekannt und bspw. in der US 2004/017928 A1 beschrieben. Der Infiltrant kann sonstige Farbmittel insbesondere zur Herstellung verschiedener Zahnfarben enthalten. Der Infiltrant kann die Farbe wechselnde Farbstoffe enthalten, die die infiltrierte Läsion anzeigen und durch Farbumschlag die Aushärtung des Infiltranten anzeigen. Bevorzugt wird der Farbstoff nach Aushärtung des Infiltranten farblos. Der Farbstoff kann radikalisch reaktiv sein.

Der Farbumschlag kann auch von anderen Einflüssen abhängen bspw. vom pH-Wert.

Der Farbstoff kann adsorptive Eigenschaften, insbesondere bezüglich des Zahnschmelzes aufweisen, so dass er sich in der oberen Schicht der Läsion anreichert. Der Farbumschlag kann dann auch im Zwischenzahnbereich besser gesehen werden.

Der Farbstoff kann nicht-adsorptive Eigenschaften aufweisen und tief in die Läsion penetrieren, so dass bspw. die Durchdringung besser kontrolliert werden kann.

Der Infiltrant kann thermo- und/oder photochrome Zusätze enthalten, durch die der infiltrierte Bereich bei Bestrahlung mit entsprechendem Licht bzw. Temperaturänderung angezeigt wird.

Offenbart ist ferner die Verwendung eines erfindungsgemäßen Infiltranten zur Behandlung und/oder Prävention von kariösen Schmelzläsionen. Eine solche Verwendung kann die nachfolgenden Schritte umfassen:

1. Entfernen einer dünnen Oberflächenschicht der Schmelzläsion durch Ätzmittel

2. Abspülen des Ätzmittels

3. Trocknen der Läsion mit einem Trocknungsmittel

4. Infiltration der Läsion mit einem Infiltranten

5. Entfernen von Überschüssen (optional)

6. Härten des Infiltranten

7. Infiltration der Läsion mit einem Infiltrant (optional)

8. Entfernen von Überschüssen (optional)

9. Härten des Infiltranten (optional)

10. Politur der infiltrierten Läsionsoberfläche (optional)

Bei einzelnen Schritten der Infiltrationsmethode kann das gewünschte Ergebnis durch Anwendung von Schall und/oder Ultraschall weiter verbessert werden, insbesondere hinsichtlich enthaltener Füllstoffe.

Bevorzugte Ätzmittel sind Gele starker Säuren wie Salzsäure.

Bevorzugte Trocknungsmittel sind toxikologisch unbedenkliche Lösungsmittel mit hohem Dampfdruck. Diese sind bspw. ausgewählt aus Alkoholen, Ketonen, Ethern, Estern usw. Besonders bevorzugt ist Ethanol.

Das Trocknungsmittel kann Bestandteile des Initiatorsystems enthalten, die nach dessen Verdunstung in der Läsion verbleiben.

Das Trocknungsmittel kann einen Filmbildner enthalten.

Nach einer ersten und/oder zweiten Infiltration kann optional ein Füllstoffe enthaltender Versiegler oder Lack aufgetragen werden, der bevorzugt einen Penetrationskoeffizienten unter 50 aufweist, mit dem Infiltranten kompatibel ist, mit diesem zusammen oder separat ausgehärtet wird und einen guten Verbund herstellt. Der Versiegler oder Lack enthält bevorzugt die erfindungsgemäßen röntgenopaken nanoskaligen Füllstoffe, bevorzugt in höheren Gehalten als der Infiltrant. Der Versiegler oder Lack kann jedoch auch andere Füllstoffe aufweisen bspw. barium- oder strontiumhaltige inerte Dentalgläser und/oder Ionomergläser.

Füllstoffe enthaltende Infiltranten können ebenfalls als Fissurenversiegler Verwendung finden.

Der Infiltrant kann antibakterielle Zusätze enthalten. Diese können bakteriostatisch, bakterizid und/oder antibiotisch wirken. Beispiele sind mit Silber ausgestattete Nanopartikel und/oder Mischoxidpartikel, Zinkoxidnanopartikel, Mischoxidpartikel, Zinn- sowie Zinkfluoride oder auch Benzalkonium, Chlorhexidin oder Triclosan.

Die Erfindung umfasst weiterhin einen Kit zur Durchführung des Infiltrationsverfahrens. Dieser umfasst

1. Ätzmittel

2. Trocknungsmittel

3. Infiltrant.

Im folgenden ist die Erfindung anhand einiger Beispiele erläutert.

Eingesetzte Komponenten

| TEDMA | Triethlenglycoldimethacrylat |
|---|---|
| E3HDDA | Ethoxyliertes 1,6-Hexandioldiacrylat |
| CQ | Kampherchinon |
| EHA | Ethylhexyl-p-N,N-dimethylaminobenzoat |
| BHT | 2,6-Di-tert.-butylphenol |
| Ph3Bi | Triphenylbismut |
| YbF3 | Ytterbiumtrifluorid (d50 ~ 40 nm) |

Prüfungsmethoden

Röntgenopazität

Die Bestimmung der Röntgenopazität geschah nach den Vorgaben der EN ISO 4049:2000 (Füllungs-, restaurative und Befestigungskunststoffe) hierfür. Aus den Infiltranten wurden durch Belichtung mit einer Halogenlampe (Heraflash; Heraeus Kulzer) etwa 1 mm dicke Probekörper hergestellt. Die exakte Probendicke wurde mittels Schieblehre festgestellt. Die Probekörper wurden zusammen mit einem Aluminiumstufenkeil (Reinheitsgrad >98% Aluminium, mit weniger als 0,1% Kupfer- und weniger als 1% Eisenanteil) auf einen Röntgenfilm (Zahnfilm Ultraspeed DF-50, Filmempfindlichkeit D, Fa. Kodak) gestellt. Probekörper, Aluminiumstufenkeil und Film wurden im Abstand von 400 mm mit einem analogen einphasigen Röntgengerät der Firma Gendex für 0,4s mit Röntgenstrahlen einer Beschleunigungsspannung von 65 kV bestrahlt. Nach dem Entwickeln und Fixieren des Films wurden die Schwärzungsgrade der Abbildungen der Probekörper und des Aluminiumstufenkeiles gemessen, eine Schwärzungskurve (Schwärzungsgrad gegen Aluminiumstufenhöhe) für den Aluminiumstufenkeil erstellt und die Werte der Röntgenopazitäten für jeden Probekörper graphisch ermittelt.

Aushärtung

Mit jeweils etwa 0,3g initiatorhaltigem Infiltrant bzw. Adper Scotchbond SE wurden zylindrische Teflonformen (5 x 10 mm hoch) gefüllt und mittels LED-Lampe (Satelec Mini-LED, Acteon, max. 2000 mW/mm$^2$) für 60 s von oben belichtet. Anschließend wurde mit einem Spatel kontrolliert, ob die Testsubstanzen ausgehärtet waren.

Oberflächenspannung

Die Oberflächenspannung der Infiltranten wurde mittels Konturanalyse eines hängenden Tropfens durchgeführt (DSA 10, KRÜSS GmbH). Die Messung der Oberflächenspannung wurde an neu gebildeten Tropfen über einen Zeitraum von 30 s durchgeführt, wobei etwa alle 5 s ein Messwert aufgenommen wurde. Hierzu wurden die Harze mit einer feinen Spritze ausgebracht und der sich bildende Tropfen mit einer Digitalkamera gefilmt. Aus der charakteristischen Form und Größe des Tropfens wurde die Oberflächenspannung gemäß der Young-Laplace-Gleichung bestimmt. Für jedes Harz wurden so 3 Messungen durchgeführt, deren Mittelwert als Oberflächenspannung angegeben wurde.

Dichtebestimmung

Die Dichten der Infiltranten wurden mittels Pyknometer bestimmt. Hierzu wurde die Dichte der Luft mit 0,0013 g/ml und die Erdbeschleunigung mit 9,8100 m/s$^2$ berücksichtigt.

Kontaktwinkel

Für jede Einzelmessung wurde Schmelz aus Rinderzähnen verwendet. Hierzu wurden Rinderzähne in einem Kunstharz eingebettet und die Schmelzfläche mittels Schleifmaschine (Struers GmbH) mit Schleifpapieren (80, 500 und 1200 Körnung) nass poliert, so dass plane etwa 0,5x1,0 cm große Schmelzflächen für die Kontaktwinkel-Messungen zur Verfügung standen. Die Schmelzproben wurden bis zur Messung in destilliertem Wasser gelagert und vor der Messung mit Ethanol und Druckluft getrocknet.
Die Messung des Kontaktwinkels erfolgte mit Hilfe eines Video-Kontaktwinkelmessgerät (DSA, KRÜSS GmbH). Hierbei wurde mittels Mikroliterspritze ein Tropfen des Infiltranten auf die Schmelzfläche gebracht, innerhalb von 10 s bis zu 40 Einzelaufnahmen des Tropfens rechnergesteuert aufgenommen und per Tropfenkonturanalyse-Software der Kontaktwinkel ermittelt.

Dynamische Viskosität

Die Viskosität der Harze wurde bei 23°C mittels dynamischen Platte-Platte-Viskosimeter (Dynamic Stress Rheometer, Rheometric Scientific Inc.) gemessen. Es wurde im "Steady Stress Sweep"-Modus bei Spaltgrößen von 0,1 bis 0,5 mm im Bereich von 0 bis 50 Pa Schubspannung ohne Vorscherung der Harze gemessen.

Beispiele

Es wurden 5 Beispielinfiltranten hergestellt und Penetrationskoeffizient (PK) und Röntgenopazität derer sowie eines Referenzmaterials bestimmt. Als Referenz wurde Adper™ Scotchbond™ SE (3M ESPE AG, Charge: 70-2010-5417-1), ein Nanofüller enthaltendes Dentaladhäsiv, verwendet. Die Zusammensetzung der Infiltranten ist in Tabelle 1 angegeben. Penetrationskoeffizient (PK) und Röntgenopazität sind in Tabelle 2 angegeben.

Tabelle 1

| Gew.-% | Vergleichsbeispiel | erfindungsgemäße Beispiele | | | |
|---|---|---|---|---|---|
| | | 1 | 2 | 3 | 4 |
| TEDMA | 80 | 80 | 80 | 80 | 80 |
| E3HDDA | 20 | 20 | 20 | 20 | 20 |
| YbF3 | 0 | 10 | 20 | 0 | 10 |
| Ph3B | 0 | 0 | 0 | 25 | 12,5 |

Zur Herstellung der Infiltranten wurden zunächst die in Tabelle 1 angegebenen Mengen der Monomere TEDMA und E3HDDA verrührt. Die angegebenen Mengen Ph3Bi wurden portionsweise zugegeben und mittels Laborrührer Monomermischung gelöst. YbF3 wurde mit einem Dispermat (VMA Getzmann, Dispergier-Scheibendurchmesser 2 cm, 2000 Umdrehungen) 30 Minuten dispergiert, so dass eine homogene Dispersion vorlag. Zum Aushärten wurde den Infiltranten 0,5 Gew.-% CQ, 0,84 Gew.-% EHA und 0,002 Gew.-% BHT zugesetzt. Die Mischungen wurden dann nochmals homogenisiert.

Tabelle 2:

| | Referenz* | Vergleichsbeispiel | erfindungsgemäße Beispiele | | | |
|---|---|---|---|---|---|---|
| | | | 1 | 2 | 3 | 4 |
| DSR-Viskosität [mPas] | 370 | 9,7 | 10,9 | 13,8 | 12,8 | 13,7 |
| Oberflächenspannnung [mN/m] | 33 | 35,6 | 34,9 | 34,9 | 36,2 | 35,3 |
| Kontaktwinkel [°] | 5,1 | 2 | 2,5 | 2,6 | 2,4 | 2,3 |
| PK [cm/s] | 4 | 183 | 160 | 126 | 141 | 139 |
| Röntgenopazität [%] | 117 | 10 | 113 | 148 | 122 | 118 |
| Aushärtung - 60 s Belichtung | ja | ja | ja | ja | ja | ja |
| *3M ESPE-Angaben im MSDS zum Adper Scotchbond SE Liquid B: 15-25% oberflächenmodifiziertes Zirkondioxid; 15-25% TEDMA, 10-15% Di-HEMA-Phosphat; 5-10% Mischung aus methacrylathaltigen Phosphorsäureestern, 5-15% Trimethylolpropantrimethacrylat, 1-10% Urethandimethacrylat. | | | | | | |

Das Referenzmaterial weist eine hohe Röntgenopazität auf, ist aufgrund des sehr niedrigen PK aber nicht oder sehr schlecht für die Infiltration von Schmelzläsionen geeignet (bspw. um tief genug in eine Schmelzläsionen zu penetrieren).

Der Infiltrant des Vergleichsbeispiels ist aufgrund des hohen PK besonders gut für die Infiltration von Schmelzläsionen geeignet. Er weist jedoch eine sehr niedrige Röntgenopazität auf, eine mit diesem infiltrierte Läsion ist im Röntgenbild nicht von einer unbehandelten Läsion zu unterscheiden.

Die erfindungsgemäßen Infiltranten der Beispiele 1-4 sind aufgrund des hohen PK gut für die Infiltration von Schmelzläsionen geeignet, zusätzlich weisen sie auch eine hohe Röntgenopazität auf, die es anhand eines Röntgenbilds ermöglicht gut zwischen infiltriertem Bereichen und Kariesläsion zu unterscheiden.

**Patentansprüche**

1. Infiltrant für die Dentalapplikation, der vernetzende Monomere und Initiator enthält und einen Penetrationskoeffizienten PK > 50 cm/s aufweist, **dadurch gekennzeichnet, dass** der Infiltrant mindestens einen nanoskaligen röntgenopaken Füllstoff aufweist, wobei der Penetrationskoeffizient wie folgt definiert ist:

$$PK = \left( \frac{\gamma . \cos\theta}{2\eta} \right)$$

mit:

$\gamma$ Oberflächenspannung des flüssigen Harzes (gegen Luft),
$\theta$ Kontaktwinkel des flüssigen Harzes (gegen Zahnschmelz),
$\eta$ dynamische Viskosität des flüssigen Harzes gemessen bei 23°C mittels dynamischem Platte-Platte-Viskosimeter.

2. Infiltrant nach Anspruch 1, **dadurch gekennzeichnet, dass** er eine Röntgenopazität nach ISO 4049 von mindestens 50 % Aluminium aufweist.

3. Infiltrant nach einem der vorangehenden Ansprüche, **dadurch gekennzeichnet, dass** es sich bei den nanoskaligen röntgenopaken Füllstoffen um Metall-, Halbmetall- oder Mischmetalloxide, -silikate, -nitride, -sulfate, - titanate, -zirkonate, -stannate, -wolframate, - phosphate, -halogenide oder um eine Mischung aus diesen Verbindungen handelt, wobei die Füllstoffe vorzugsweise ausgewählt sind aus der Gruppe bestehend aus Aluminiumoxid, Zirkondioxid, Titandioxid, Zinkoxid, Zinndioxid, Ceroxid, Aluminium-Silizium-Oxide, Silizium-Zink-Oxide, Silizium-Zirkon-Oxide, Eisenoxide und deren Mischungen mit Siliziumdioxid, Indiumoxide und deren Mischungen mit Siliziumdioxid und/oder Zinndioxid, Siliziumdioxid, Bornitrid, Strontiumsulfat, Bariumsulfat, Strontiumtitanat, Bariumtitanat, Natriumzirkonat, Kaliumzirkonat, Magnesiumzirkonat, Calciumzirkonat, Strontiumzirkonat, Bariumzirkonat, Natriumwolframat, Kaliumwolframat, Magnesiumwolframat, Calciumwolframat, Strontiumwolframat und/oder Bariumwolframat.

4. Infiltrant nach Anspruch 1 oder 2, **dadurch gekennzeichnet, dass** die nanoskaligen röntgenopaken Füllstoffe Salze, einschließlich schwerlösliche Salze, vorzugsweise Sulfate, Phosphate oder Fluoride der Seltenerdmetalle,

- vorzugsweise ausgewählt aus der Gruppe bestehend aus Lanthan, Cer, Samarium, Gadolinium, Dysprosium, Erbium oder Ytterbium,
- des Scandiums, des Yttriums, des Bariums und Strontiums, oder Wolframaten, vorzugsweise Orthowolframate, sind, wobei die nanoskaligen röntgenopaken Füllstoffsalze, vorzugsweise Wolframate, vorzugsweise mit Metallatomen mindestens eines Metalls dotiert sind und wobei vorzugsweise das Wirtsgittermetall des Füllstoffsalzes durch den Dotierungsstoff in einer Menge von bis zu 50 Mol-%, weiter bevorzugt 0,1 bis 40 Mol-%, besonders bevorzugt 0,5 bis 30 Mol-%, insbesondere 1 bis 25 Mol-% ersetzt ist.

5. Infiltrant nach einem der vorangehenden Ansprüche, **dadurch gekennzeichnet, dass** der röntgenopake nanoskalige Füllstoff eine mittlere Primärpartikelgröße $d_{50}$

- kleiner 1000 nm, kleiner 700 nm, kleiner 500 nm, kleiner 200 nm, kleiner 100 nm, kleiner 25 nm,
- zwischen 1 nm und 80 nm, zwischen 4 nm und 60 nm, zwischen 6 nm und 50 nm, zwischen 0,5 nm und 22 nm, zwischen 1 nm und 20 nm, zwischen 1 nm und 10 nm oder zwischen 1 nm und 5 nm aufweist, wobei die röntgenopaken nanoskaligen Füllstoffe vorzugsweise vereinzelt vorliegende, nicht aggregierte und nicht agglomerierte nanoskalige Füllstoffe, besonders bevorzugt mit unimodaler Teilchengrößenverteilung umfassen.

6. Infiltrant nach einem der vorangehenden Ansprüche, **dadurch gekennzeichnet, dass** der röntgenopake nanoskalige Füllstoff eine BET-Oberfläche gemäß DIN 66131 bzw. DIN ISO 9277 zwischen 15 m$^2$/g und 600 m$^2$/g, bevorzugt zwischen 30 m$^2$/g und 500 m$^2$/g und besonders bevorzugt zwischen 50 m$^2$/g und 400 m$^2$/g aufweist.

7. Infiltrant nach einem der vorangehenden Ansprüche, **dadurch gekennzeichnet, dass** der Gehalt des nanoskaligen röntgenopaken Füllstoffs im Infiltranten bezogen auf die Gesamtmasse des Infiltranten mit allen Inhaltsstoffen 1 Gew.-% bis 30 Gew.-%, vorzugsweise 5 Gew.-% bis 25 Gew.-%, weiter vorzugsweise 10 Gew.-% bis 20 Gew.-% oder 1 Gew.-% bis 5 Gew.-%, 5 Gew.-% bis 10 Gew.-%, 10 Gew.-% bis 15 Gew.-% oder 15 Gew.-% bis 20 Gew.-% beträgt.

8. Infiltrant nach einem der vorangehenden Ansprüche, **dadurch gekennzeichnet, dass** der röntgenopake nanoskalige Füllstoff organisch modifiziert ist, vorzugsweise durch Behandeln mit einem Siloxan, Chlorsilan, Silazan, Titanat, Zirkonat, Wolframat oder mit einer organischen Säure, einem organischen Säurechlorid oder -anhydrid, wobei die Siloxane, Chlorsilane, Silazane, Titanate, Zirkonate und Wolframate bevorzugt folgende allgemeine Formeln aufweisen:

- $Si(OR')_n R_{4-n}$,
- $SiCl_n R_{4-n}$,
- $(R_m R''_{3-m} Si)_2 NH$,
- $Ti(OR')_n R_{4-n}$,
- $Zr(OR')_n R_{4-n}$ und
- $W(OR')_n R_{6-n}$,

wobei

- m = 1, 2 oder 3 ist,
- n = 1, 2 oder 3, vorzugsweise n gleich 3 ist,
- die über den Sauerstoff gebundene Gruppe R' und die Gruppe R" eine beliebige organische funktionelle Gruppe, vorzugsweise eine Alkylgruppe, besonders bevorzugt eine Methyl-, Ethyl-, Propyl- oder Isopropylgruppe ist und
- die funktionelle Gruppe R eine beliebige organische Gruppe und direkt über ein Kohlenstoffatom an das Silizium, Titan, Zirkon oder Wolfram gebunden ist.

9. Infiltrant nach einem der vorangehenden Ansprüche, **dadurch gekennzeichnet, dass** er einen Penetrationskoeffizienten PK > 100 cm/s aufweist.

10. Infiltrant nach einem der vorangehenden Ansprüche, **dadurch gekennzeichnet, dass** er gemessen bei 23°C mittels dynamischem Platte-Platte-Viskosimeter eine dynamische Viskosität von 50 mPas oder weniger, vorzugsweise 30 mPas oder weniger, weiter vorzugsweise 15 mPas oder weniger aufweist.

11. Kit zur Herstellung eines Infiltranten nach einem der vorangehenden Ansprüche, **dadurch gekennzeichnet, dass** der Kit

- eine erste Komponente mit Monomeren und chemisch aktivierbaren Initiatoren und
- eine zweite Komponente mit Aktivatoren enthält und
- optional zusätzlich Ätz- und/oder Trocknungsmittel aufweist.

12. Infiltrant nach einem der Ansprüche 1 bis 10 zur Behandlung und/oder Prävention

- von kariösen Schmelzläsionen und/oder
- zur Verwendung als Fissurenversiegler.

**Claims**

1. An infiltrant for dental application which contains crosslinking monomers and initiator and has a penetration coefficient PC of > 50 cm/s, **characterised in that** the infiltrant comprises at least one nanoscale x-ray opaque filler, wherein the penetration coefficient is defined as follows:

$$PC = \left( \frac{\gamma \cdot cos\theta}{2\eta} \right)$$

where:

$\gamma$ surface tension of the liquid resin (relative to air),
$\theta$ contact angle of the liquid resin (relative to dental enamel),

$\eta$ dynamic viscosity of the liquid resin measured at 23°C by means of a dynamic plate-plate viscometer.

2. An infiltrant according to claim 1, **characterised in that** it has an X-ray opacity to ISO 4049 of at least 50% aluminium.

3. An infiltrant according to any one of the preceding claims, **characterised in that** the nanoscale X-ray opaque fillers are metal, semimetal or mischmetal oxides, silicates, nitrides, sulfates, titanates, zirconates, stannates, tungstates, phosphates, halides or a mixture of these compounds, wherein the fillers are preferably selected from the group consisting of aluminium oxide, zirconium dioxide, titanium dioxide, zinc oxide, tin dioxide, cerium oxide, aluminium-silicon oxides, silicon-zinc oxides, silicon-zirconium oxides, iron oxides and mixtures thereof with silicon dioxide, indium oxides and mixtures thereof with silicon dioxide and/or tin dioxide, silicon dioxide, boron nitride, strontium sulfate, barium sulfate, strontium titanate, barium titanate, sodium zirconate, potassium zirconate, magnesium zirconate, calcium zirconate, strontium zirconate, barium zirconate, sodium tungstate, potassium tungstate, magnesium tungstate, calcium tungstate, strontium tungstate and/or barium tungstate.

4. An infiltrant according to claim 1 or claim 2, **characterised in that** the nanoscale X-ray opaque fillers are salts, including sparingly soluble salts, preferably sulfates, phosphates or fluorides of rare earth metals,

   - preferably selected from the group consisting of lanthanum, cerium, samarium, gadolinium, dysprosium, erbium or ytterbium,
   - of scandium, of yttrium, of barium and strontium, or tungstates, preferably orthotungstates, wherein the nanoscale X-ray opaque filler salts, preferably tungstates, are preferably doped with metal atoms of at least one metal and wherein preferably the host lattice metal of the filler salt is replaced by the dopant in a quantity of up to 50 mol%, more preferably 0.1 to 40 mol%, particularly preferably 0.5 to 30 mol%, in particular 1 to 25 mol%.

5. An infiltrant according to any one of the preceding claims, **characterised in that** the X-ray opaque nanoscale filler has an average primary particle size $d_{50}$

   - of smaller than 1000 nm, smaller than 700 nm, smaller than 500 nm, smaller than 200 nm, smaller than 100 nm, smaller than 25 nm,
   - between 1 nm and 80 nm, between 4 nm and 60 nm, between 6 nm and 50 nm, between 0.5 nm and 22 nm, between 1 nm and 20 nm, between 1 nm and 10 nm or between 1 nm and 5 nm,
   wherein the X-ray opaque nanoscale fillers preferably comprise individually present, non-aggregated and non-agglomerated nanoscale fillers, particularly preferably with a unimodal particle size distribution.

6. An infiltrant according to any one of the preceding claims, **characterised in that** the X-ray opaque nanoscale filler has a BET surface area to DIN 66131 or DIN ISO 9277 of between 15 $m^2$/g and 600 $m^2$/g, preferably between 30 $m^2$/g and 500 $m^2$/g and particularly preferably between 50 $m^2$/g and 400 $m^2$/g.

7. An infiltrant according to any one of the preceding claims, **characterised in that** the content of the nanoscale X-ray opaque filler in the infiltrant relative to the total mass of the infiltrant with all components amounts to 1 wt.% to 30 wt.%, preferably 5 wt.% to 25 wt.%, more preferably 10 wt.% to 20 wt.% or 1 wt.% to 5 wt.%, 5 wt.% to 10 wt.%, 10 wt.% to 15 wt.% or 15 wt.% to 20 wt.%.

8. An infiltrant according to any one of the preceding claims, **characterised in that** the X-ray opaque nanoscale filler is organically modified, preferably by treatment with a siloxane, chlorosilane, silazane, titanate, zirconate, tungstate or with an organic acid, an organic acid chloride or anhydride, wherein the siloxanes, chlorosilanes, silazanes, titanates, zirconates and tungstates preferably have the following general formulae:

   - $Si(OR')_n R_{4-n}$,
   - $SiCl_n R_{4-n}$,
   - $(R_m R''_{3-m} Si)_2 NH$,
   - $Ti(OR')_n R_{4-n}$,
   - $Zr(OR')R_{4-n}$ and
   - $W(OR')_n R_{6-n}$,

wherein

   - m = 1, 2 or 3,

- n = 1, 2 or 3, preferably n equals 3,
- the group R' attached via the oxygen and the group R" is any desired organic functional group, preferably an alkyl group, particularly preferably a methyl, ethyl, propyl or isopropyl group and
- the functional group R is any desired organic group and is directly attached via a carbon atom to the silicon, titanium, zirconium or tungsten.

9. An infiltrant according to any one of the preceding claims, **characterised in that** it has a penetration coefficient PC of > 100 cm/s.

10. An infiltrant according to any one of the preceding claims, **characterised in that** it has a dynamic viscosity, measured at 23°C by means of a dynamic plate-plate viscometer, of 50 mPa·s or lower, preferably 30 mPa·s or lower, more preferably 15 mPa·s or lower.

11. A kit for producing an infiltrant according to any one of the preceding claims, **characterised in that** the kit contains

   - a first component with monomers and chemically activatable initiators and
   - a second component with activators and
   - optionally additionally comprises etchant and/or desiccant.

12. An infiltrant according to any one of claims 1 to 10 for the treatment and/or prevention

   - of carious enamel lesions and/or
   - for use as a fissure sealant.

**Revendications**

1. Agent infiltrant pour applications dentaires, qui contient des monomères réticulables et un amorceur, et présente un coefficient de pénétration PK > 50 cm/s, **caractérisé en ce que** l'agent infiltrant comprend au moins une charge radio-opaque nanoscalaire, le coefficient de pénétration étant défini comme suit :

$$PK = \left(\frac{\gamma.\cos\theta}{2\eta}\right)$$

dans laquelle

   $\gamma$ est la tension superficielle de la résine liquide (par rapport à l'air),
   $\theta$ est l'angle de contact de la résine liquide (par rapport à l'émail dentaire),
   $\eta$ est la viscosité dynamique de la résine liquide, mesurée à 23°C à l'aide d'un viscosimètre dynamique à disques parallèles.

2. Agent infiltrant selon la revendication 1, **caractérisé en ce qu'**il présente une radio-opacité selon ISO 4049 d'au moins 50 % aluminium.

3. Agent infiltrant selon l'une des revendications précédentes, **caractérisé en ce que**, pour ce qui concerne les charges radio-opaques nanoscalaires, il s'agit d'oxydes, de silicates, de nitrures, de sulfates, de titanates, de zirconates, de stannates, de tungstates, de phosphates d'halogénures de métaux, de métalloïdes ou de métaux mixtes, ou d'un mélange de ces composés, les charges étant de préférence choisies dans le groupe consistant en l'oxyde d'aluminium, le dioxyde de zirconium, le dioxyde de titane, l'oxyde de zinc, le dioxyde d'étain, l'oxyde de cérium, les oxydes d'aluminium et de silicium, les oxydes de silicium et de zinc, les oxydes de silicium et de zirconium, les oxydes de fer et les mélanges de ceux-ci avec le dioxyde de silicium, les oxydes d'indium et les mélanges de ceux-ci avec le dioxyde de silicium et/ou le dioxyde d'étain, le dioxyde de silicium, le nitrure de bore, le sulfate de strontium, le sulfate de baryum, le titanate de strontium, le titanate de baryum, le zirconate de sodium, le zirconate de potassium, le zirconate de magnésium, le zirconate de calcium, le zirconate de strontium, le zirconate de baryum, le tungstate de sodium, le tungstate de potassium, le tungstate de magnésium, le tungstate de calcium, le tungstate de strontium et/ou les tungstates de baryum.

**4.** Agent infiltrant selon la revendication 1 ou 2, **caractérisé en ce que** les charges radio-opaques nanoscalaires sont des sels, notamment des sels difficilement solubles, de préférence des sulfates, des phosphates ou des fluorures des métaux des terres rares,

- de préférence choisis dans le groupe consistant en le lanthane, le cérium, le samarium, le gadolinium, le dysprosium, l'erbium ou l'ytterbium,
- du scandium, de l'yttrium, du baryum et du strontium, ou des tungstates, de préférence des orthotungstates, les sels formant charges radio-opaques nanoscalaires, de préférence les tungstates, étant dopés de préférence par des atomes métalliques d'au moins un métal, et de préférence le métal du réseau hôte du sel formant charge étant remplacé par la substance dopante en une quantité allant jusqu'à 50 % en moles, d'une manière plus préférée de 0,1 à 40 % en moles, d'une manière particulièrement préférée de 0,5 à 30 % en moles, en particulier de 1 à 25 % en moles.

**5.** Agent infiltrant selon l'une des revendications précédentes, **caractérisé en ce que** la charge nanoscalaire radio-opaque présente une granulométrie primaire moyenne $D_{50}$

- inférieure à 1000 nm, inférieure à 700 nm, inférieure à 500 nm, inférieure à 200 nm, inférieure à 100 nm, inférieure à 25 nm,
- comprise entre 1 nm et 80 nm, entre 4 nm et 60 nm, entre 6 nm et 50 nm, entre 0,5 nm et 22 nm, entre 1 nm et 20 nm, entre 1 nm et 10 nm ou entre 1 nm et 5 nm,
les charges nanoscalaires radio-opaques comprenant des charges se présentant de préférence sous forme individualisée, non agrégées et non agglomérées, présentant de préférence une distribution granulométrique unimodale.

**6.** Agent infiltrant selon l'une des revendications précédentes, **caractérisé en ce que** la charge nanoscalaire radio-opaque présente une aire BET selon DIN 66131 ou DIN ISO 9277 comprise entre 15 $m^2$/g et 600 $m^2$/g, de préférence entre 30 $m^2$/g et 500 $m^2$/g et d'une manière particulièrement préférée entre 50 $m^2$/g et 400 $m^2$/g.

**7.** Agent infiltrant selon l'une des revendications précédentes, **caractérisé en ce que** la teneur de l'agent infiltrant en la charge radio-opaque nanoscalaire, rapportée à la masse totale de l'agent infiltrant, avec tous ses constituants, est de 1 % en poids à 30 % en poids, de préférence de 5 % en poids à 25 % en poids, d'une manière plus préférée de 10 % en poids à 20 % en poids ou de 1 % en poids à 5 % en poids, de 5 % en poids à 10 % en poids, de 10 % en poids à 15 % en poids ou de 15 % en poids à 20 % en poids.

**8.** Agent infiltrant selon l'une des revendications précédentes, **caractérisé en ce que** la charge nanoscalaire radio-opaque a subi une modification organique, de préférence par traitement avec un siloxane, un chlorosilane, un silazane, un titanate, zirconate, un tungstate, ou avec un acide organique, un chlorure d'acyle ou un anhydride d'acide organique, les siloxanes, les chlorosilanes, les silazanes, les titanates, les zirconates et les tungstates ayant de préférence les formules générales suivantes :

- $Si(OR')_n R_{4-n}$,
- $SiCl_n R_{4-n}$,
- $(R_m R''_{3-m} Si)_2 NH$,
- $Ti(OR')_n R_{4-n}$,
- $Zr(OR')_n R_{4-n}$ et
- $W(OR')_n R_{6-n}$,

dans lesquelles

- $m = 1, 2,$ ou $3$,
- $n = 1, 2$ ou $3$, et de préférence $n$ est égal à $3$,
- le groupe R' lié par l'intermédiaire de l'oxygène et le groupe R'' sont des groupes fonctionnels organiques quelconques, de préférence des groupes alkyle, d'une manière particulièrement préférée les groupes méthyle, éthyle, propyle ou isopropyle, et
- le groupe fonctionnel R est un groupe organique quelconque et est directement lié par un atome de carbone au silicium, au titane, au zirconium ou au tungstène.

**9.** Agent infiltrant selon l'une des revendications précédentes, **caractérisé en ce qu'**il présente un coefficient de pénétration PK > 100 cm/s.

**10.** Agent infiltrant selon l'une des revendications précédentes, **caractérisé en ce qu'**il présente, mesurée à 23°C à l'aide d'un viscosimètre dynamique à disques parallèles, une viscosité dynamique de 50 mPa.s ou moins, de préférence de 30 mPa.s ou moins, d'une manière plus préférée de 15 mPa.s ou moins.

**11.** Trousse pour fabriquer un agent infiltrant selon l'une des revendications précédentes, **caractérisée en ce que** la trousse contient

      - un premier composant, comportant des monomères et des amorceurs chimiquement activables, et
      - un deuxième composant comportant des activateurs, et
      - en option comprend en outre un agent caustique et/ou un dessiccant.

**12.** Agent infiltrant selon l'une des revendications 1 à 10 pour le traitement et/ou la prévention

      - de lésions carieuses de l'émail et/ou
      - pour utilisation en tant que résine pour scellement de puits et fissures.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007131725 A1
- DE 19638068 A1
- DE 202008006814
- EP 1570831 A1
- EP 1872767 A
- EP 1720506 A1
- WO 2007131725 A
- US 6387981 A
- EP 1413569 A
- EP 1741419 A
- EP 1688125 A
- WO 2005086911 A
- WO 02066535 A
- WO 2005121200 A
- WO 02062901 A
- WO 2006031972 A
- EP 1714633 A
- EP 1285947 A
- EP 1849450 A
- US 2004017928 A1


**In der Beschreibung aufgeführte Nicht-Patentliteratur**

- **BUCKTON G.** Interfacial phenomena in drug delivery and targeting. *Chur,* 1995
- **FAN P. L. et al.** Penetrativity of sealants. *J. Dent. Res.,* 1975, vol. 54, 262-264
- Römpp Chemie Lexikon. Georg Thieme Verlag, 1990, 1711